(19) Europäisches Patentamt
European Patent Office
Office européen des brevets

(11) Veröffentlichungsnummer: **0 134 485**
**A2**

(12) # EUROPÄISCHE PATENTANMELDUNG

(21) Anmeldenummer: 84108002.1

(22) Anmeldetag: 09.07.84

(51) Int. Cl.[4]: C 07 D 491/056
A 61 K 31/47
//C07D317/62, C07D317/68,
(C07D491/056, 317/00, 221/00)

(30) Priorität: 20.07.83 DE 3326190

(43) Veröffentlichungstag der Anmeldung:
20.03.85 Patentblatt 85/12

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Grohe, Klaus, Dr.
Am Wasserturm 10
D-5068 Odenthal(DE)

(72) Erfinder: Zeiler, Hans-Joachim, Dr.
Elsbeekerstrasse 46
D-5620 Velbert 15(DE)

(72) Erfinder: Metzger, Karl Georg, Dr.
Pahlkestrasse 75
-D-5600 Wuppertal 1(DE)

(54) **5-Cyclopropyl-5,8-dihydro-8-oxo-1,3-dioxolo-(4,5-g) chinolin-7-carbonsäure, Verfahren zu ihrer Herstellung sowie diese enthaltende antibakerielle Mittel.**

(57) Die neue 5-Cyclopropyl-5,8-dihydro-8-oxo-1,3-dioxolo[4,5-g]-chinolin-7-carbonsäure ist antibakteriell wirksam und soll als Arzneimittel verwendet werden. Sie wird in einer mehrstufigen Synthese hergestellt.

EP 0 134 485 A2

Croydon Printing Company Ltd

0134485

BAYER AKTIENGESELLSCHAFT 5090 Leverkusen, Bayerwerk

Si/Ke-c

Konzernverwaltung RP
Patentabteilung

5-Cyclopropyl-5,8-dihydro-8-oxo-1,3-dioxolo-[4,5-g] chinolin-7-carbonsäure, Verfahren zu ihrer Herstellung sowie diese enthaltende antibakterielle Mittel

Die vorliegende Erfindung betrifft die neue 5-Cyclopropyl-5,8-dihydro-8-oxo-1,3-dioxolo[4,5-g]chinolin-7-carbonsäure, Verfahren zu ihrer Herstellung sowie diese enthaltende antibakterielle Mittel.

Gefunden wurde die neue 5-Cyclopropyl-5,8-dihydro-8-oxo-1,3-dioxolo[4,5-g]chinolin-7-carbonsäure der Formel (I)

O COOH N (I)

und deren pharmazeutisch verwendbaren Salze und Hydrate.

Sie eignet sich als Wirkstoff für die Human- und Veterinärmedizin, wobei zur Veterinärmedizin auch die Vorbeugung und Behandlung bei Fischen zu zählen ist.

Die 5-Cyclopropyl-5,8-dihydro-8-oxo-1,3-dioxolo/4,5-g/chinolin-7-carbonsäure (I) kann gemäß folgendem Reaktionsschema hergestellt werden:

COOH
Cl
(1)

$SOCl_2$

COCl
Cl
(2)

$CH_2(COOC_2H_5)_2$
$Mg(OEt)_2$

$-CO-CH(COOC_2H_5)_2$
Cl
(3)

$-C(=O)-CH_2COOC_2H_5$
Cl
(4)

$-C(=O)-C(COOC_2H_5)=CH-OC_2H_5$
Cl
(5)

$-C(=O)-C(COOC_2H_5)=CH-HN$-Cyclopropyl
Cl
(6)

$COOC_2H_5$
(7)

COOH
(I)

Danach wird Malonsäurediethylester in Gegenwart von Magnesiumethylat mit 6-Chlor-piperonylsäurechlorid (2) zum Aroylmalonester (3) acyliert (Organicum, 3. Auf. 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäureethylester (4), der mit o-Ameisensäuretriethylester/Acetanhydrid in den substituierten 3-Ethoxyacrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol führt in leicht exothermer Reaktion zum gewünschten Zwischenprodukt (6).

Die Cyclisierungsreaktion (6) $\rightarrow$ (7) wird in einem Temperaturbereich von etwa 60 bis 300°C, bevorzugt 80 bis 180°C durchgeführt.

Als Verdünnungsmittel können Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstufe Kalium-tert.-butanolat, Butyl-lithium, Lithium-phenyl, Phenylmagnesiumbromid. Natriummethylat, Natriumhydrid und besonders bevorzugt Kalium- oder Natriumcarbonat in Betracht. Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zur 5-Cyclopropyl-5,8-dihydro-8-oxo-1,3-dioxolo/4,5-g/chinolin-7-carbonsäure (I).

Das als Ausgangsmaterial für diesen Syntheseweg verwendete 6-Chlor-piperonylsäurechlorid (2) wurde aus der literaturbekannten 6-Chlor-piperonylsäure (1) (A.M. Bleakly Orr, R. Robinson und M. M. Williams, J. Chem. Soc. (London) 111, 946 (1917)) mit Thionylchlorid auf übliche Weise erhalten. Es besitzt einen Siedepunkt von 136 bis 141°C/0,12 mbar und einen Schmelzpunkt von 77 bis 78°C.

Als neuer Wirkstoff seien die 5-Cyclopropyl-5,8-dihydro-8-oxo-1,3-dioxolo/4,5-g/chinolin-7-carbonsäure und deren pharmazeutisch verwendbaren Alkalisalze, Erdalkalisalze oder Hydrate genannt.

Herstellung von I

A) 6-Chlor-piperonylsäurechlorid (2)

68,5 g 6-Chlor-piperonylsäure (1) werden in rascher Tropfenfolge mit 250 ml Thionylchlorid versetzt. Man erhitzt bis zur Beendigung der Gasentwicklung unter Rückfluß zum Sieden (ca. 2 Stunden) und destilliert das überschüssige Thionylchlorid bei Normaldruck ab. Der Rückstand wird i. Feinvak. fraktioniert. Bei 136 bis 141°C/0,12 mbar gehen 65,5 g (2) vom Schmelzpunkt 77 bis 78°C über.

B) 6-Chlor-piperonyl-malonsäurediethylester (3)

7,3 g Magnesiumspäne werden in 15 ml wasserfreiem Ethanol suspendiert. Man versetzt mit 1,5 ml Tetrachlorkohlenstoff und tropft, wenn die Reaktion in Gang gekommen ist, ein Gemisch von 48 g Malonsäurediethylester, 30 ml abs. Ethanol und 120 ml wasserfreiem Toluol bei 50 bis 60°C zu. Dann wird noch 1 Stunde auf 50 bis 60°C erhitzt, mit Trockeneis/Aceton auf -5°C bis -10°C gekühlt und bei dieser Temperatur eine Lösung von 65,5 g 6-Chlor-piperonylsäurechlorid (2) in 200 ml abs. Toluol langsam zugetropft. Man rührt 1 stunde bei 0 bis -5°C, läßt über Nacht auf Raumtemperatur kommen und läßt unter Eiskühlung ein Gemisch von 120 ml Eiswasser und 18 ml konz. Schwefelsäure zulaufen. Die Phasen werden getrennt und dreimal mit Toluol nachextrahiert. Die vereinigten Toluollösungen werden mit gesättigter NaCl-Lösung gewaschen, mit $Na_2SO_4$ getrocknet und das Lösungsmittel im Vakuum abgezogen. Man erhält 100,8 g 6-Chlor-piperonyl-malonsäurediethylester (3) als Rohprodukt.

C) 6-Chlor-piperonyl-essigsäureethylester (4)

Eine Emulsion von 100,8 g rohem 6-Chlor-piperonyl-malonsäurediethylester (3) in 150 ml Wasser wird mit 0,1 g p-Toluolsulfonsäure versetzt. Man erhitzt unter gutem Rühren 3,5 Stunden zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel im Vakuum ab. Die Fraktionierung des Rückstandes

im Feinvakuum liefert 48,5 g 6-Chlor-piperonyl-essigsäureethylester (4) vom Siedepunkt 170 bis 193°C/0,14 bis 0,3 mbar.

D) 3-Ethoxy-2-(6-chlor-piperonyl)-acrylsäureethylester (5)

Ein Gemisch von 46,3 g 6-Chlor-piperonyl-essigsäureethylester (4), 37,7 g o-Ameisensäureethylester und 42 g Acetanhydrid wird 2 Stunden auf 150°C erhitzt. Dann werden im Wasserstrahlvakuum und zuletzt im Feinvakuum bei einer Badtemperatur von ~120°C die flüchtigen Bestandteile abdestilliert. Zurück bleiben 53,6 g roher 3-Ethoxy-acrylsäureethylester (5). Er ist genügend rein für die weiteren Umsetzungen.

E) 3-Cyclopropylamino-2-(6-chlor-piperonyl)-acrylsäure-ethylester (6)

Eine Lösung von 32,6 g 3-Ethoxyacrylsäureethylester (5) in 100 ml Ethanol wird unter Eiskühlung und Rühren tropfenweise mit 6 g Cyclopropylamin versetzt. Wenn die exotherme Reaktion abgeklungen ist, wird noch 1 Stunde bei Raumtemperatur gerührt, mit 100 ml Wasser versetzt, nach Eiskühlung abgesaugt und mit $H_2O$/Ethanol 1 : 1 gewaschen. Man erhält 26,8 g 3-Cyclopropylamino-2-(6-chlor-piperonyl)-acrylsäureethylester (6) vom Schmelzpunkt 149 bis 151°C.

F) 5-Cyclopropyl-5,8-dihydro-8-oxo-1,3-dioxolo[4,5-g]chinolin-7-carbonsäureethylester (7)

Eine Lösung von 26,6 g 3-Cyclopropylamino-acrylsäure-

ethylester (6) in 100 ml wasserfreiem Dimethylformamid wird mit 13 g Kaliumcarbonat versetzt. Dann wird 2 Stunden unter Rückfluß gerührt und das Reaktionsgemisch heiß auf Eis gegossen. Der Niederschlag wird abgesaugt, mit Wasser gut gewaschen und im Vakuum über Calciumchlorid bei 100°C getrocknet. Man erhält 20,6 g 5-Cyclopropyl-5,8-dihydro-8-oxo-1,3-dioxolo[4,5-g]chinolin-7-carbonsäureethylester (7) vom Schmelzpunkt 225 bis 227°C.

G) 5-Cyclopropyl-5,8-dihydro-8-oxo-1,3-dioxolo[4,5-g]chinolin-7-carbonsäure (I)

Ein Gemisch von 9 g 5-Cyclopropyl-5,8 dihydro-8-oxo-1,3-dioxolo[4,5-g]chinolin-7-carbonsäureethylester (7), 120 ml Wasser und 2,2 g Aetzkali wird 2 Stunden unter Rückfluß erhitzt. Die Lösung wird bei Raumtemperatur filtriert, mit 10-proz. Salzsäure auf pH 1 angesäuert, der Niederschlag abgesaugt, mit Wasser gewaschen und im Vakuum bei 120°C getrocknet. Man erhält auf diese Weise 8 g 5-Cyclopropyl-5,8-dihydro-8-oxo-1,3-dioxolo[4,5-g]chinolin-7-carbonsäure (I) vom Zersetzungspunkt 316 bis 320 °C.

Die erfindungsgemäße Verbindung zeigt bei geringer Toxizität ein breites antibakterielles Spektrum gegen grampositive und gram-negative Keime, insbesondere gegen Enterobaktereaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglicht ihre Verwendung als chemotherapeutischen Wirkstoff in der Medizin sowie als Stoff zur Konservierung von anorganischen und organischen Materialien, insbesondere von organischen Materialien aller art, z.B. Polymeren, Schmiermittel, Farben, Fasern, Leder, Papier und Holz, von Lebensmitteln und von Wasser.

Die erfindungsgemäße Verbindung ist gegen ein sehr breites Spektrum von Mikroorganismen wirksam. Mit ihrer Hilfe können gram-negative und gram-positive Bakterien und bakterienähnliche Mikrooranismen bekämpft sowie die durch diese Erreger hervorgerufenen Erkrankungen verhindert, gebessert und/oder geheilt werden.

Besonders wirksam ist die erfindungsgemäße Verbindung gegen Bakterien und bakterienähnliche Mikroorganismen. Sie ist daher besonders gut zur Prophylaxe und Chemotherapie von lokalen und systemischen Infektionen in der Human- und Tiermedizin geeignet die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, (Staph. = Staphylococcus); Lactobacteriaceae, wie Streptokokken, z.B. Streptococcus

pyogenes, α- bzw. ß-hämolysierende Streptokokken, nicht (γ-)hämolysierende Streptokokken, Enterokokken und Diplococcus pneumoniae (Pneumokokken)
Enterobacteriaceae, wie Escherichiae-Bakterien der Coli-Gruppe: Escherichia-Bakterien, z.B. Escherichia coli, Enterobacter-Bakterien, z.B. Eaerogenes, E. Cloacae, Klebsiella-Bakterien, z.B. K. pneumoniae, Serratia, z.B. Serratia marcescens (E. = Enterobacter) (K. = Klebsiella), Proteae-Bakterien der Proteus-Gruppe: Proteus, z.B. Proteus vulgaris, Pr. morganii, Pr. rettgeri, Pr. mirabilis (Pr. = Proteus);

Pseudomonadaceae, wie Pseudomonas-Bakterien, z.B. Pseudomonas aeruginosa.

Bacteriodaceae, wie Bacteroides-Bakterien, z.B. Bacteroides fragilis; Mykoplasmen, z. B. Mykoplasma pneumonia.

Die obige Aufzählung von Erregern ist lediglich beispielhaft und keineswegs beschränkend aufzufassen.

Als Krankheiten, die durch die erfindungsgemäße Verbindung verhindert, gebessert und/oder geheilt werden können, seien beispielsweise genannt:

Otitis; Pharyngitis; Pneumonie, Peritonitis; Pyelonephritis; Cystitis; Endocarditis; Systeminfektionen; Bronchitis; Arthritis; lokale Infektionen, septische Erkrankungen.

Zur vorliegenden Erfindung gehören pharmazeutische Zubereitungen, die neben nicht-toxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können

den Wirkstoff neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den übliche, gegebenenfalls Opakisierungsmittel enthaltenden, Überzügen und Hüllen versehen sein und auch so zusammengesetzt sein, daß sie den Wirkstoff nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der Wirkstoff kann gegebenenfalls mit einem oder mehreren der oben angegebenen Trägerstoffe auch in mikroverkapselter Form vorliegen.

Suppositorien können neben dem Wirkstoff die üblichen wasserlöslichen oder wasserunlöslichen Trägerstoffe enthalten, z.B. Polyethylenglykole, Fette, z.B. Kakaofett

und höhere Ester (z.B. $C_{14}$-Alkohol mit $C_{16}$-Fettsäure) oder Gemische dieser Stoffe.

Salben, Pasten, Cremes und Gele können neben dem Wirkstoff die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem Wirkstoff die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel, z.B. Chlorfluorkohlenwasserstoffe, enthalten.

Lösungen und Emulsionen können neben dem Wirkstoff die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteraler Applikation können die Lösungen und Emulsionen auch in steriler und blutisotonischer Form vorliegen.

Suspensionen können neben dem Wirkstoff die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydrid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Sußmittel, z.B. Saccharin, enthalten.

Die therapeutisch wirksame Verbindung soll in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer der erfindungsgemäßen Verbindung auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des Wirkstoffes mit dem oder den Trägerstoffen.

Der Wirkstoff oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den erfindungsgemäßen Wirkstoff, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart des Wirkstoffes kann durch jeden Fachmann aufgrund seines Fachwissens leicht erfolgen.

Die neue Verbindung kann in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte der erfindungsgemäßen Verbindung angegeben.

Tabelle

| Stamm | MHK mcg/ml<br>I |
|---|---|
| E. coli Neumann | <0,015 |
| E. coli 4418 | <0,015 |
| E. coli 455/7 | 16 |
| Klebsiella 63 | <0,015 |
| Klebsiella 6179 | 0,06 |
| Klebsiella 8085 | <0,015 |
| Proteus mir. 8223 | 8 |
| Proteus 8175 | 0,03 |
| Serratia 16040 | 4 |
| Staph. aur. 133 | 0,25 |
| Streptococc. faec. 9790 | 8 |
| Pseudom. W. | 4 |

Agarverdünnungstest
Isosensitest-Medium
Denley Multipoint-Inokulator

Patentansprüche

1. 5-Cyclopropyl-5,8-dihydro-8-oxo-1,3-dioxolo/4,5-g/chinolin-7-carbonsäure der Formel (I)

(I)

und deren pharmazeutisch verwendbaren Salze und Hydrate.

2. 5-Cyclopropyl-5,8-dihydro-8-oxo-1,3-dioxolo/4,5-g/chinolin-7-carbonsäure der Formel (I)

(I)

und deren pharmazeutisch verwendbaren Salze und Hydrate bei der Behandlung bakterieller Erkrankungen bei Menschen und/oder Tieren.

3. Verfahren zur Herstellung der Verbindung der Formel (I) in Anspruch 1, dadurch gekennzeichnet, daß man 6-Chlor-piperonylsäurechlorid mit Malonsäurediethylester zum entsprechenden Aroylmalonsäurediethylester acyliert und diesen partiell verseift

und decarboxyliert; den entstehenden Aroylessigsäureethylester mit Orthoameisensäuretriethylester kondensiert zum entsprechenden 3-Ethoxy-acrylsäureethylester, diesen mit Cyclopropylamin umsetzt, das entstehende Produkt bei 60 bis 300°C, vorzugsweise 80 bis 180°C, cyclisiert und den erhaltenen Ester verseift.

4. Arzneimittel, gekennzeichnet durch einen Gehalt an mindestens einer Verbindung der Formel (I) in Anspruch 1.

5. Verwendung von Verbindungen der Formel (I) in Anspruch 1 bei der Bekämpfung von bakteriellen Erkrankungen.

6. Verfahren zur Herstellung von antibakteriellen Arzneimitteln, dadurch gekennzeichnet, daß man Verbindungen der Formel (I) in Anspruch 1 mit inerten, nichttoxischen, pharmazeutisch geeigneten Trägerstoffen vermischt.